# EUROPEAN PATENT SPECIFICATION

(11) **EP 0 325 768 B1**
(45) Date of publication and mention of the grant of the patent: **26.04.1995**
(21) Application number: 88121231.0
(22) Date of filing: 19.12.1988
(51) Int. Cl.: C12N 15/00, A61K 39/13

(54) **Non-reverting rna viruses**
Nichtzurückschlagende RNS-Viren
Virus à ARN non retournant

(30) Priority: 11.01.1988 US 142507
(43) Date of publication of application: 02.08.1989
(73) Proprietor: AMERICAN CYANAMID COMPANY, Wayne, NJ 07470-8426 (US)
(72) Inventor: Weeks-Levy, Carolyn Louise, Thornwood New York 10954 (US); Mento, Steven Joseph, Tenafly New Jersey 07670 (US)
(74) Representative: Wächtershäuser, Günter, Prof. Dr.

(56) References cited:
- EP-A- 0 156 299
- WO-A-82/03632
- CHEMICAL ABSTRACTS, vol. 104, no. 11, MARCH 1986, page 173, ref. no. 83080r Columbus, Ohio, US; & JP-A-60 207 582 (NIPPON POLIO RESEARCH INSTITUTE FOUNDATION) 19-10-85
- BIOLOGICAL ABSTRACTS, vol. 84, no. 11, 1987, page AB-369, ref. no. 107609, Philadelphia, US; N. LA MONICA et al.: "A mouse model for poliovirus neurovirulence identifies mutations that attenuate the virus for humans"
- SCIENCE, vol. 214, November 20, 1981, pages 916-1919; V.R. RACANIELLO et al.: "Cloned poliovirus complementary DNA is infectious in mammalian cells"
- BIOLOGOCAL ABSTRACTS, vol. 83, no. 7, 1987, page AB-421, ref. no. 64538 Philadelphia, US; V.R. RACANIELLO et al.: Poliovirus temperature sensitive mutant containing a single nucleotide deletion in the 5-noncoding region of the viral RNA" & VIROLOGY 155(2): 498-507, 1986
- PROCEEDINGS OF THE NATIONAL ACADEMY OF SCIENCES OF THE USA; vol. 82, no. 24, December 1985, pages 8424-8428, Washington, US G. Kaplan et al.: "In vitro synthesis of infectious poliovirus RNA"
- PROCEEDINGS OF THE NATIONAL ACADEMY OF SCIENCES OF THE USA, vol. 78, no. 8, August 1981, pages 4887-4891, Washington, US V:R:RACANIELLO et al.: Molecular cloning of poliovirus cDNA and determination of the complete nucleotide sequence of the viral genome"
- J. MOL. BIOL. vol. 174, 1984, pages 561-585, Academic Press Inc., London, GB; H: TOyoda et al: Complete nucleotide sequences of all three poliovirus serotype genomes. Implication for genetic relationship, gene function and antigenic determinants"

## Description

### TECHNICAL FIELD OF THE INVENTION

This invention relates to attenuated RNA viral strains having a low rate of replication. More particularly this invention relates to vaccines and the use of such vaccines comprising those attenuated RNA viral strains.

### BACKGROUND OF THE INVENTION

Various positive strand RNA viruses or picornaviruses are well known. Typical picornaviruses include polioviruses, echoviruses, coxsackieviruses and rhinovirus (common cold viruses).

Vaccines, to protect against diseases induced by such viruses are prepared using live or killed viral varients or mutants that are attenuated. Attenuated viruses exhibit a lowered pathogenicity. For example, Albert Sabin in the 1950's isolated attenuated strains of the poliovirus by passing the virus in tissues from different animal hosts in cultured cells, until varients were selected,having lost their capacity to cause disease [A. J. Sabin et al., "History of Sabin Attenuated Poliovirus Oral Live Vaccine Strains", J. Biol. Stard., 1, p. 115 (1973)]. Virus based vaccines are useful because they are able to replicate and induce adequate immunity through the production of antibodies. However, live vaccines have the additional benefit of inducing immunity at the site of infection whereas killed vaccines are limited to inducing serum immunity. Live virus vaccines are commonly distributed because of the ease of oral administration, low cost and the rapidity vaccine recipients develop long-lasting immunity. Unfortunately, the live viruses may revert, i.e., from the live viral pool may emerge new varients that are capable of replicating to cause disease, either once in the vaccine recipient or during manufacture.

N. LaMonica et al., "A Mouse Model for Poliovirus Neurovirulence Identifies Mutations That Attenuate the Virus for Humans", J.Virology, 61, pp. 2917-2920 (1987), discusses the potential for development of vaccines containing mutagenized viruses so as to reduce the rate of reversion to the virulent form."

It would therefore be of interest to produce a strain that is less likely to revert for use as a vaccine.

### SUMMARY OF THE INVENTION

The present invention solves the problem referred to above by providing highly attentuated RNA viral strains that have a lowered potential of reverting to virulence. RNA viral strains of this invention are useful in vaccines to protect recipients or those in close contact from viral induced disease.

One embodiment of a process of this invention for producing these attenuated RNA viral strains is the modification of a RNA viral genome to increase the base pairing of the viral genome to itself.

A second embodiment of a process of this invention is the modification of a RNA viral genome, in order to increase or decrease base pairing between the viral RNA and mammalian host ribosomal RNA. The process of this invention modifies viral RNA bases in regions containing complementarity with host ribosomal RNA. A third embodiment of a process of this invention is the combination of the above two embodiments to provide highly attenuated RNA viral strains.

Such modifications of base pairing comprise the steps of isolating RNA viruses, reverse transcribing the RNA to give cDNA copies; mutagenizing the cDNA to cause one or both of the effects referred to above in the two embodiments; transfecting cells capable of producing viral RNA with the mutagenized RNA viral cDNA; and, culturing to produce attenuated RNA virus. These modifications are effective to reduce the transcriptional or translational efficiency of RNA viral strains.

An object of this invention is to employ the attenuated RNA viruses in a live vaccine. Administration of the vaccine may be via any of the conventional accepted modes of administering live vaccines.

### BRIEF DESCRIPTION OF THE DRAWINGS

Figure 1 depicts a loop predicted by matching-up complementary bases from the published cDNA polio genome between bases 370 and 471 of the 5′ noncoding region [Toyoda et al., J. Mol. Biol., 174, p. 561 (1984)], and the resulting modified loop after mutagenesis.

Figure 2 is a schematic depiction of the 7500 b.p. poliovirus genome having complementarity with cellular mouse ribosomal RNA.

Figure 3 depicts the interaction of published 28S rat ribosomal sequences [Genbank, "NucleotideSequences", Vol. 1, p. 243, Oxford Press (1985)] with polio cDNA sequences (Toyoda et al., supra).

### DETAILED DESCRIPTION OF THE INVENTION

The present invention relates to a process for the production of highly attenuated RNA viral strains. Attenuated RNA viral strains, as defined by this invention, have a lowered transcriptional or translational efficiency which results in slower replication of viruses. Because the generation of new viral populations is reduced, there is a less likelihood of producing a viral varient that causes disease.

As described above, in the first embodiment of this invention, the viral RNA genome has been altered by increasing the base pairing to itself. While not wishing to be bound by theory, we believe an increase in base pairing stabilizes viral RNA folding, which in turn makes it more difficult to unwind for initiation of transcription of translation. We also believe modified base pairing involves changes the secondary structure of the viral RNA, which disrupts its binding to host ribosomal RNA during transcription or translation. These disruptions reduce the efficiency of transcription or translation, hence viral production decreases. Accordingly, viral strains modified by the processes of this invention are characterized by a reduced rate of replication as compared with the native or other unmodified strains. A more attenuated strain results because the rate of producing any potential viral varient that may induce disease is lowered. The viral RNA that is modified, is located in the looped regions of the genome. In these loops, caused by base-base interactions, there are unbound base residues. We choose to substitute according to the method described below, unbound bases with bases having complementarity with another unbound base.

Also, as described above as another embodiment of this invention, the viral RNA genome is modified in regions containing complementarity with host ribosomal RNA to provide highly attenuated viruses. Modifications of this sort includes increasing or decreasing base pairing between the viral RNA and host ribosomal RNA. We believe such base-base alteration also disrupts the efficiency of translation, which leads to lower production of any potential viral variant.

The invention also relates to a process for producing highly attenuated RNA viruses of this invention. In the preferred embodiment of this process we purify viruses in order to remove any cellular debris, followed by synthesizing full length viral double stranded cDNa according to the method of Racaniello et al. [Y. R. Racaniello et al., "Molecular Cloning of Poliovirus cDNA And Determination Of The Complete Nucleotide Sequence Of the Viral Genome", Proc. Natl. Acad. Sci. U.S.A., 78, p. 4887 (1981)]. Next, according to the first embodiment of this invention, RNA viral cDNA that correspond to the RNA bases that were unbound in the RNA viral genome looped structure are mutagenized using conventional techniques to increase base pairing. Also, according to the second embodiment of this invention, the RNA viral cDNA corresponding to regions of the viral RNA containing complementarity with host ribosomal RNA is mutagenized either to increase or decrease the base pairing between the RNA genome and the ribosomal RNA. After mutagenesis cells capable of RNA production, for example CV-l, VERO, or Hela cells, are transfected with cDNA, using conventional techniques. Live virus is next detected, using known methods and sequenced by known techniques to determine if the RNA viral sequence corresponds to the mutagenized cDNA sequence. Finally, the degree of attenuation is detected. We use an assay measuring the rate of production of viral protein labelled with 35S-methionine.

The RNA viral strains of this invention can be employed in a vaccine using known methods in produce live vaccines. The resulting vaccine will contain an amount of live virus effective in the recipient to protect against viral infection. Administration of those vaccines may be via any of the conventional accepted modes of administration of live vaccines. In order that this invention may be better understood, the following example is set forth. This example is for the purpose of illustration only and is not to be construed as limiting the scope of the invention.

### EXAMPLE

We chose to illustrate this invention using rat ribosomal RNA as a host and polio viral RNA as a model. The preferred host and corresponding ribosomal RNA is human. However, because of the highly conserved homology among eukaryotic ribosomal RNA, one skilled in the art could use a rat host to exemplify the teachings of this invention.

### 1. Virus Purification

We used polioviruses (available from the F.D.A.) maintained in culture conditions described by A. Sabin et al., "Studies on Varients of Poliomyetits Virus", J. Exp. Med., 99:551 (1954). Specifically we infected primary monkey kidney tissue at a low multiplicity of infection and incubated the virus at 34° at pH 7.3. To remove any cellular debris we centrifuged harvest fluid containing the strain at low speed (2000 RPM for 20 minutes). We precipitated the virus by adding NaCl (.12M) and polyethylene glycol 8000 (12% by weight) followed by stirring overnight at 4°C. The precipitated virus was collected by centrifugation at 10,000 RPM for 20 minutes.

After resuspending the virus pellet in a total volume of 1.2 ml of TNE (10 mM Tris-HCl, 100 mM NaCl, 1 mM EDTA, at pH 8.0), we added NONIDET P-40 until the final concentration was 1% by weight. We next layed the virus onto 15%-45% sucrose gradients [P. P. Minor, "Comparative Biochemical Studies of Type 3 Polio Virus", J. Virol, 34, p. 73 (1980)], and spun them at 12,300 RPM for 16 hours in a SW 28.1 rotor. We collected 1 ml. fractions from the top of the gradient. Twenty-five microliter samples were taken from each sample and were run on discontinuous 15% polyacrylamide gels. We located the poliovirus in the fractions by visualization of the capsid proteins in the gel by silver straining the gels [W. Wray et al., "Silver Staining of Proteins In Polyacrylamide Gels", Analy. Bioch., 118, p. 197 (1981)]. Sucrose fractions containing poliovirus were pooled and sodium dodecyl sulfate (SDS) was added to a final concentration of 0.5%. We next extracted virus from the pooled sucrose fractions with an equal volume of phenol containing chloroform and isoamyl alcohol (25:24:1) once, and back extracted the phenol once. Aqueous layers were pooled and 0.5 volumes of 7.5M ammonium acetate was added. Using 2.5 volumes of absolute ethanol and freezing at -70°C overnight poliovirus RNA was precipitated. Afterwards, we resuspended the RNA pellet in water for injection (WF1) that had been previously treated with diethyl pyrocarbonate (DEP) and reprecipitated the RNA by adding 2.5 volumes of absolute ethanol. After repelling the RNA and washing twice with ice cold 70% ethanol, the final RNA pellet was resuspended in 20 ul of DEP treated WFI. We next sequence the RNA according to the method of Sanger [Sanger et al., "DNA Sequencing With Chain Terminating Inhibitors", Proc. Nat'l. Acad. Sci. USA, 74, p. 5463 (1973)] with modifications for RNA and problems with secondary structure associated with RNA templates [D. C. DeBorde et al., "Resolution Of A Common RNA Sequencing Ambiguity By Terminal Deoxynucleotidyl Transferase", Analy. Bioch., 157, p. 275 (1986)]. For example, terminal deoxynucleotidyl transferase is used to eliminate artifacts due to secondary structure.

### 2. Synthesis of Full Length Poliovirus Double-Stranded cDNA

RNA from the previous step is used as a template for the synthesis of double stranded cDNA Racaniello et al., supra. Using oligo(dg).oligo(dc) tailing method of Bothwell et al. [A. L. M. Bothwell, Cell, 24, p. 625 (1981)], double stranded cDNA are inserted into the PstI site of plasmid pBR322. Tetracycline-resistant clones are screened for inserts by colony hybridization, using poliovirus cDNA probe [M. Grunstein et al., Proc. Nat'l. Acad. Sci USA, 72, p. 3961 (1975)]. Using nucleotide sequence analysis and restriction enzyme mapping techniques the inserts are aligned on the viral genome (V. R. Racanicello et al. infra). Finally, partial poliovirus cDNA clones are ligated into a single, full length clone, at their common cleavage sites [Racanicello et al., "Cloned Poliovirus cDNA Is Infectious In Mammalian Cells", Science, 214, p. 916 (1981)].

### 3. Mutagenesis of Poliovirus cDNA

In accordance with the first embodiment of this invention, poliovirus cDNA from the previous step corresponding to unbound bases in the looped RNA genome are mutagenized to increase base pairing with other unbound bases located within the loop or outside of the loop in the linear portion of the genome.

As a means for mutagenizing unbound base pairs, site-directed mutagenesis of poliovirus cDNA is employed using the Ml3 method [C. A. Hutchinson III et al., "Mutagenesis At A Specific Position In A DNA Sequence", J. Biol. Chem., 253, p. 655 (1978); A. Razin et al., "Efficient Correction Of A Mutation By Use Of A Chemically Synthesized DNA", Proc. Natl. Acad. Sci. USA, 75, p. 4268 (1978)].

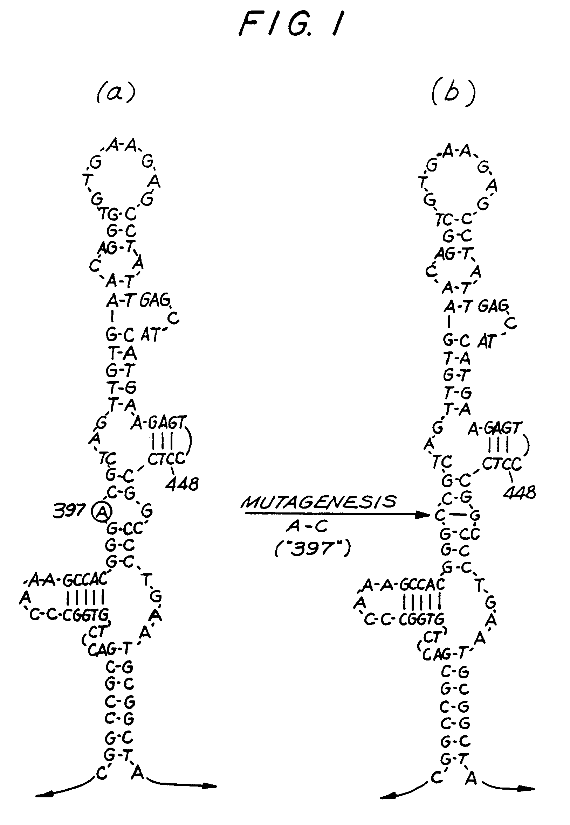

Fig. 1 depicts a cDNA loop of the polio genome with unbound bases. To increase base pairing the adenine base at position "397" is substituted by a cytosine base in order to bind to the unbound quanine (See Fig. 1.). Other unbound bases can be substituted in this manner in order to increase base pairing.

In accordance with the second embodiment of this invention, we mutagenize poliovirus cDNA corresponding to the regions containing complementarity with rat ribosomal RNA, by either increasing or decreasing base pairing between the poliovirus genome and rat ribosomal RNA.

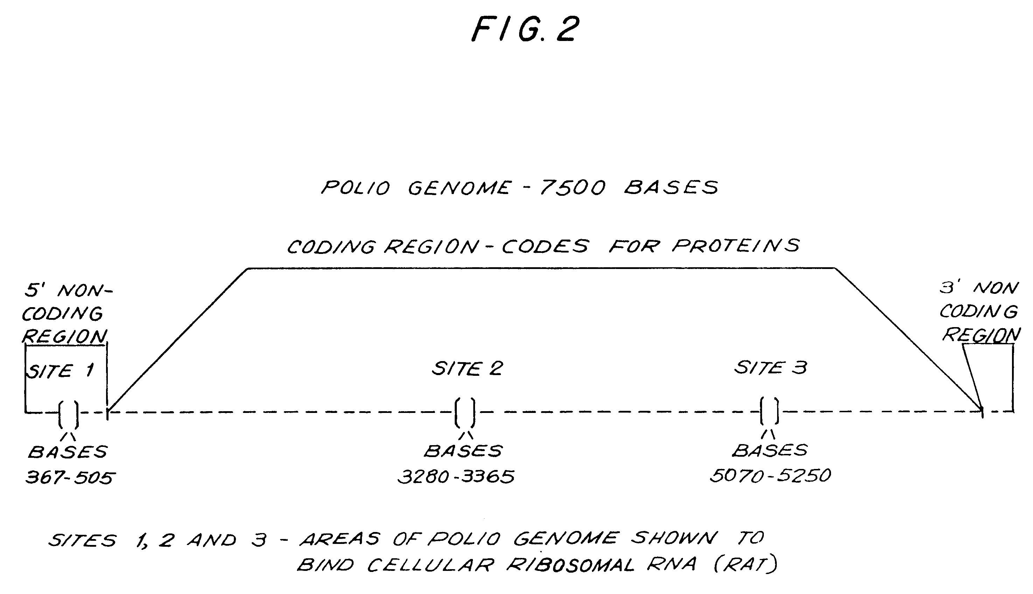

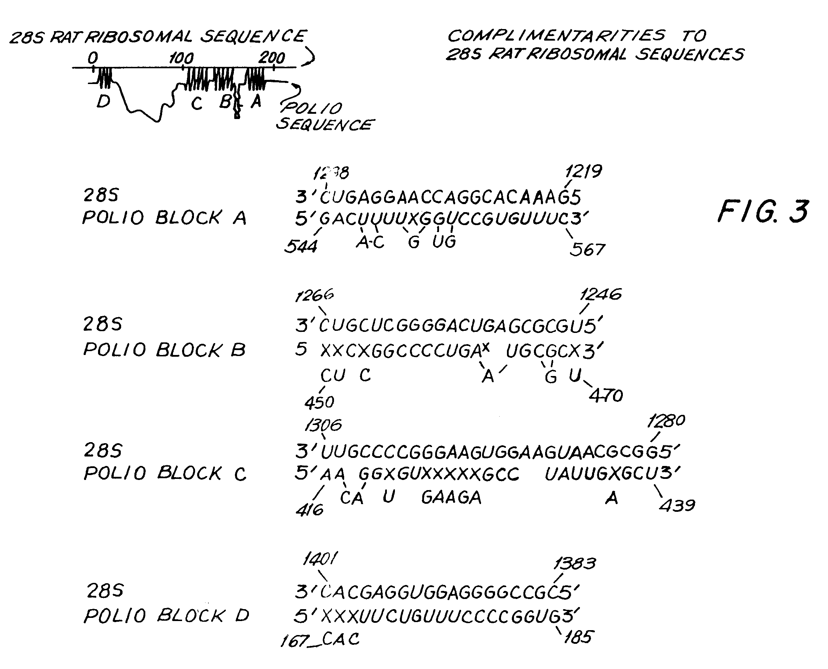

As shown in Figure 2, areas of the polio genome have been shown to bind with cellular rat RNA. Within these areas are sequences that have complementarity with rat ribosomal RNA (see Fig. 3). Base residues within these sequences are mutagenized by the above method to either increase or decrease base pairing with the rat ribosomal RNA.

### 4. Transfection Of Cells With Mutagenized Poliovirus Clone

Having mutagenized poliovirus cDNA, host cells capable of RNA production, for example, VERD or HeLa cells, are transfected with plasmid cDNA. Transfection is conducted using a calcium-phosphate technique [B. A. Parker, J. Virol., 31, p. 360 (1979)], modified by treating the host cells with electroportion to help them take up this cDNA (apparatus from Promega, Madison, Wisconsin).

### 5. Detection Of Live Poliovirus

After transfection of the mutagenized polio cDNA, production of virus is detected by looking for the active production of poliovirus by cell lysis, also called cytopathic effect (CPE) [T. H. Donnebacker, "Correlation Of The Stage Of Cytopathic Change With The Release Of Poliomyelitis Virus", Virology, 2, p. 399 (1956)]. To confirm that the RNA viral sequence corresponds to the mutagenized cDNA sequence, the RNA genome is sequenced according to the method of Sanger et al., infra.

### 6. Determination Of The Degree Of Attenuation

The rate of production of viral protein is used as an assay for attenuation, comparing the currently used Sabin strain and the strain of this invention. viral protein is labelled in the presence of ³⁵S methionine and the protein products are separated by SDS-polyacrylamide electrophoresis, followed by autoradiographing and scanning the gel to determine the different levels of viral proteins and the degree of inhibition of host protein synthesis. Confirmation that the decrease in translational and efficiency results in decreased neurovirulance is determined by comparing existing vaccine strains to newly made strains, in accordance with this invention. in a monkey neurovirulence test (21 CFR 600-799, 1987).

### 7. Preparation Of The Live Vaccine

After assaying for attenuation the viral particle selected now becomes the master seed from which other colonies are derived. For example the master seed is infected to Vero cells on microcarriers contained in a bioreactor, harvested and purified for use in a vaccine.

While we have hereinbefore described a number of embodiments of this invention, it is apparent that our basic constructions can be altered to provide other embodiments which utilize the processes and compositions of this invention.

## Claims

1. A method for producing modified highly attenuated RNA viral strains **characterized** by having a reduced rate of replication in a mammalian host, said method comprising:
a) isolating RNA viral sequences from viral particles;
b) preparing RNA viral cDNA from said RNA viral sequences;
c) mutagenizing said RNA viral cDNA to increase base pairing of the cDNA to itself such that said mutagenesis reduces transcriptional and translational efficiency of RNA viral strains produced from said RNA viral cDNA;
d) transfecting cells with said mutagenized RNA viral cDNA;
e) culturing said host cells; and
f) extracting RNA viral strains from said cells.

2. The method according to Claim 1, wherein the RNA viral strain is poliovirus.

## Patentansprüche

1. Verfahren zur Erzeugung modifizierter, hoch abgeschwächter RNA-Virusstämme, dadurch gekennzeichnet, daß sie eine verringerte Replikationsrate in einem Säugerwirt aufweisen, wobei das Verfahren umfaßt:
a) das Isolieren von RNA-Virussequenzen aus Virusteilchen;
b) das Herstellen von RNA-viraler cDNA aus den RNA-Virussequenzen;
c) das Mutagenisieren der RNA-viralen cDNA, um die Basenpaarung der cDNA mit sich selbst zu vergrößern, so daß die Mutagenese die Transkriptions- und Translationseffizienz von RNA-Virusstämmen verringert, die aus der RNA-viralen cDNA erzeugt wurden;
d) das Transfizieren von Zellen mit der mutagenisierten RNA-viralen cDNA;
e) das Kultivieren der Wirtszellen; und
f) das Extrahieren von RNA-Virusstämmen aus den Zellen.

2. Verfahren nach Anspruch 1, in dem der RNA-Virusstamm Poliovirus ist.

## Revendications

1. Procédé de préparation de souches virales à ARN modifiées considérablement atténuées caractérisées par un taux de réplication réduit dans un hôte mammifère, ledit procédé comprenant:
a) l'isolement de séquences d'ARN viral à partir des particules virales;
b) la préparation d'une copie sous forme d'ADNc de l'ARN viral desdites séquences;
c) la mutagenèse de ladite copie d'ADNc de l'ARN viral de manière à augmenter l'appariement des bases de l'ADNc à lui-même de telle sorte que ladite mutagenèse réduise l'efficacité transcriptionnelle et traductionnelle des souches virales à ARN produites à partir desdites copies sous forme d'ADNc de l'ARN viral;
d) la transfection des cellules avec lesdites copies sous forme d'ADNc de l'ARN viral mutées;
e) la culture desdites cellules hôtes; et
f) l'extraction des souches virales à ARN à partir desdites cellules.

2. Procédé selon la revendication 1, dans lequel la souche virale à ARN est le poliovirus.
